# EUROPÄISCHE PATENTSCHRIFT

(11) **EP 1 570 284 B1**
(45) Veröffentlichungstag und Bekanntmachung des Hinweises auf die Patenterteilung: **03.05.2006**
(21) Anmeldenummer: 03795770.1
(22) Anmeldetag: 12.12.2003
(51) Int. Cl.: G01R 33/28, A61B 5/055, A61F 2/01

(54) **GEFÄSSFILTER FÜR MR-BILDGEBUNGSVERFAHREN**
VASCULAR FILTER FOR MR-IMAGING METHODS
FILTRE VASCULAIRE POUR PROCEDE D'IMAGERIE PAR RESONANCE MAGNETIQUE

(30) Priorität: 12.12.2002 DE 10258708
(43) Veröffentlichungstag der Anmeldung: 07.09.2005
(73) Patentinhaber: aMRIs Patente Verwaltungs GmbH & Co. KG, 44757 Castrop-Rauxel (DE)
(72) Erfinder: MELZER, Andreas, 45481 Mülheim an der Ruhr (DE); SCHAEFERS, Gregor, 46236 Bottrop (DE)
(74) Vertreter: Weidener, Jörg Michael
(86) Internationale Anmeldenummer: PCT/DE2003/004199
(87) Internationale Veröffentlichungsnummer: WO 2004/053512

(56) Entgegenhaltungen:
- EP-A- 1 092 985
- SCHAEFERS G. ET AL: "New vena cava filter (VCF) with integrated inductively coupled resonator for MR micro imaging" ISMRM TENTH MEETING PROCEEDINGS, 18. Mai 2002 (2002-05-18), XP002277496 HONOLULU, HI, USA
- BARTELS L W ET AL: "Improved Lumen Visualization in Metallic Vascular Implants by Reducing RF Artifacts" MAGNETIC RESONANCE IN MEDICINE, WILEY, Bd. 47, Nr. 1, Januar 2002 (2002-01), Seiten 171-180, XP002277498 USA ISSN: 0740-3194
- SISKIN G.P., BARTHOLOMEW K.: "Inferior Vena Cava Filters"[Online] 6. Dezember 2002 (2002-12-06), Seiten 1-25, XP002277497 Gefunden im Internet: URL:http://www.emedicine.com/radio/topic76 2.htm> [gefunden am 2004-04-20]
- BUSCH M. ET AL: "A Physical Explanation of Active MRI Stents (aMRIs) and first "in vitro" and "in vivo" Results" ISMRM TENTH MEETING PROCEEDINGS, 18. Mai 2002 (2002-05-18), XP002277512 HONOLULU, HI, USA

## Beschreibung

Die Erfindung betrifft einen Gefäßfilter gemäß dem Oberbegriff des Anspruchs 1.

Gefäßfilter sind Strukturen, die in durchströmte Venen und Arterien des menschlichen oder tierischen Körpers, insbesondere in die Vena cava inferior, eingesetzt werden, um das Eindringen von Blutgerinnseln oder sklerotischem Gefäßmaterial in Organe des Körpers, insbesondere die Lunge und das Gehirn, zu verhindern.

Zur Applikation werden Gefäßfilter in der Regel im nichtexpandierten Zustand auf einen in den Körper einzuführenden Draht aufgesteckt, befestigt oder in einem Katheter über einen operativen Venenzugang an den Zielort der Implantation verbracht und dann entfaltet. Dabei wird der Durchmesser des Gefäßfilters vergrößert, so dass sich der Filter gegen die Gefäßwand drückt bzw. dort befestigt.

Es besteht Bedarf, den Gefäßfilter während der Implantation und zur späteren Funktionskontrolle oder zur Entfernung durch bildgebende Verfahren darzustellen. In letzter Zeit gewinnen Magnetresonanz- (MR) Bildgebungssysteme in der medizinischen Diagnostik zunehmend an Bedeutung. Insbesondere werden interventionelle und minimalinvasive Techniken wie Punktion, Katheterisierung und operative Verfahren unter MR-tomographischer Kontrolle durchgeführt. Die Darstellung und Positionierungsbestimmung eines Gefäßfilters in einem MR-Bildgebungssystem ist allerdings schwierig. So sind Gefäßfilter aus Metall nur artefaktbehaftet sichtbar und Gefäßfilter aus Kunststoffen wegen ihrer feinen Struktur im MR-Bild kaum sichtbar.

Aus der Veröffentlichung "G. Schaefers, et al, "New vena cava filter (VCF) with integrated inductively coupled reesonator for MR micro imaging", Proc. ISMRM, 10.05.2002, HI, USA;" ist ein Gefäßfilter mit mindestens einer Leiterschleife bekannt, die die Induktivität eines elektrischen Resonanzschwingkreises ausbildet. Aus der WO-A1 99/19739 ist es bekannt, für eine deutliche und signalintensive Darstellung einer medizinischen Vorrichtung im MR-Bild in diese Vorrichtung einen Schwingkreis zu integrieren, der in einem lokal begrenzten Bereich in oder um die medizinische Vorrichtung eine veränderte Signalantwort erzeugt, die ortsaufgelöst dargestellt wird. Die Resonanzfrequenz des Schwingkreises ist im wesentlichen gleich der Resonanzfrequenz der eingestrahlten Hochfrequenzstrahlung des MR-Bildgebungssystems. Der Schwingkreis und die medizinische Vorrichtung können dabei aus dem gleichen Material gefertigt sein. In einer Ausführungsform ist der Schwingkreis derart in die medizinische Vorrichtung integriert, dass sich die Induktivität beim Entfalten der Vorrichtung zusammen mit dieser auffaltet.

Aus der Schrift ebenfalls bekannt sind MR-aktive Gefäßfilter. Diese Instrumente bestehen aus einem Filteranteil und einer separaten Leiterschleife, die um den Filter gewickelt ist. Nach Einbringung in den biologischen Körper können Leiterschleife und Gefäßfilter entfaltet werden.

Nachteilig an Gefäßfiltern dieser Art ist die Tatsache, dass sie aus einem Grundgerüst für die Filterfunktion bestehen, auf die eine Leiterschleife herumgewickelt werden müss, um das Gerät mit der gewünschten MR-Aktivität zu versehen. Die Verbindung zweier Komponenten wirkt sich dabei nachteilig auf die mechanische Stabilität des Gefäßfilters aus.

Es ist die Aufgabe der vorliegenden Erfindung, einen Gefäßfilter bereitzustellen, der sich durch gute mechanische Eigenschaften, insbesondere ein hohes Maß an Flexibilität bei gleichzeitiger Stabilität auszeichnet und der dabei in einem MR-Bildgebungssystem gut darstellbar ist.

Diese Aufgabe wird erfindungsgemäß durch einen Gefäßfilter mit den Merkmalen des Anspruchs 1 gelöst. Bevorzugte und vorteilhafte Ausgestaltungen der Erfindung sind in den Unteransprüchen angegeben.

Der Gefäßfilter weist mindestens eine Leiterschleife auf, die die Induktivität eines elektrischen Resonanzschwingkreises ausbildet. Dabei besteht die Leiterschleife aus einem Stück und bildet den Gefäßfilter aus. Der erfindungsgemäße Gedanke liegt somit darin, nur eine Struktur, nämlich eine Leiterschleife, sowohl für die Ausbildung des eigentlichen Filters als auch für die Induktivität zu verwenden. In Kombination mit einer Kapazität wird somit ein Resonanzschwingkreis bereitgestellt.

In einer vorteilhaften Ausführungsform bildet der Gefäßfilter zusätzlich zu dem Resonanzschwingkreis mindestens einen integrierten Schaltkreis aus, der derart mit den Resonanzschwingkreisen gekoppelt ist, dass diese durch den integrierten Schaltkreis einstellbar oder verstimmbar sind. Der integrierte Schaltkreis ermöglicht eine externe Beeinflussung der Resonanzfrequenz mit Verstimmung oder Abstimmung, beispielsweise durch Dazuschalten und/oder Abschalten von induktiven oder kapazitiven Elementen des Schwingkreises. Die Stromversorgung des integrierten Schwingkreises kann durch eine kleine Energiequelle (z. B. Batterie) oder auch durch induktive Einkopplung von Energie aus einem elektromagnetischen Feld erfolgen.

In einer vorteilhaften Ausführungsform weist die Leiterschleife Abstandselemente und/oder Isolatoren auf, die einzelne Abschnitte der Leiterschleife in Abstand zueinander halten und/öder gegeneinander isolieren. Dadurch werden Kurzschlüsse zwischen einzelnen Leiterwindungen verhindert. Außerdem sind die Isolatoren in einer besonders vorteilhaften Ausführungsform in Verbindung mit mindestens einer Leiterschleife gleichzeitig ein Teil der internen Kapazität. Diese entsteht durch eine Verbindung und gleichzeitige Isolierung der Enden mit einem Isolierstoff (z. B. Polymer, Keramik, Verbundwerkstoff), der ein Dielektrikum darstellt und mit mindestens zwei durch den Isolierstoff getrennten Leitern mindestens eine im Netzwerk elektrische verschaltete Kapazität (Kondensator) ausbildet.

Mit Vorteil ist die Leiterschleife mit einem Nichtleiter, insbesondere Kunststoff und/oder Keramik, ummantelt. Dies dient einer vergrößerten Haltbarkeit durch vergrößerte mechanische Stabilität und einer störungsfreien Funktion des Gefäßfilters. In einer besonders bevorzugten Ausführungsform dient die Isolierung der Verminderung und Regulierung der parasitären Kapazität, wobei die Isolierung auch zum "Tunen" des Schwingkreises, also der Feineinstellung der Resonanzfrequenz, verwendet werden kann. Eine Feineinstellung ist ebenfalls durch die Verformung des Filters und somit durch eine Änderung der Induktivität möglich. Hauptsächlich kann dies durch die Expansion oder Kompression bzw. durch den limitierenden Gefäßdurchmesser bestimmt werden. Der erfindungsgemäße Gefäßfilter kommt somit in seiner einfachsten Ausführungsform ohne eine zusätzliche Kapazität aus, da eine Kombination aus interner und parasitärer Kapazität für seinen Funktion ausreicht. Auch wird durch die Isolierung wiederum sichergestellt, dass zwischen den Enden der einzelnen Leiterschleifen kein elektrischer Kontakt zu anderen Leiterschleifen vorliegt, der die vorgesehene Induktivität durch Kurzschluss verändern würde.

In einer vorteilhaften Ausführungsform des erfindungsgemäßen Gefäßfilters hat der Resonanzschwingkreis eine Resonanzfrequenz, insbesondere im hochfrequenten Bereich, die der Frequenz eines äußeren Magnetfeldes, insbesondere eines MR-Tomographen (MRT), entspricht. Damit kann die Implantation und die Funktion des Gefäßfilters mittels bildgebender MR-Verfahren beobachtet werden. Es ist jedoch ebenso denkbar, einen Schwingkreis mit einer Resonanzfrequenz in einem anderen Frequenzbereich vorzusehen.

In einer vorteilhaften Ausführungsform weist die Leiterschleife mindestens ein elektrisch nichtleitendes Material auf, auf dessen Oberfläche mindestens ein leitendes Material, insbesondere Gold, Platin, Tantal und/oder leitfähige Legierungen, aufgebracht ist. Die Wahl von besonders leitfähigen Materialien, wie z. B. Gold, verbessert dabei die Ausbildung der Resonanz. Es können auch mehrere Schichten aus Isolator und Leiter auf die Leiterschleife aufgebracht werden.

Mit Vorteil ist die Leiterschleife des Gefäßfilters so ausgebildet, dass sie entfaltbar ist. Bevorzugt kann der Resonanzschwingkreis während und/oder nach der Implantation in einen biologischen Körper ausgebildet werden. Somit kann der Gefäßfilter in gefalteter Form implantiert werden und während der Implantation oder erst an seinem Zielort entfaltet werden. Dies ermöglicht die Implantation ausgehend von kleinen Venen oder Arterien in der Peripherie des biologischen Körpers. Es ist aber grundsätzlich auch möglich, ihn vor der Transplantation auszubilden.

Unter Leiterschleife wird dabei ein aus einem Stück bestehender Leiter verstanden. Unter einer Leiterschleifenwindung wird ein Abschnitt einer Leiterschleife verstanden, der in eine bestimmte Form gebracht worden ist, wobei auch die gerade Form eingeschlossen ist.

In einer weiteren vorteilhaften Ausführungsform, dem sogenannten Umbrellafilter, weist der Gefäßfilter mehrere Leiterschleifenwindungen auf, die derart geführt sind, dass die Leiterschleife eine längliche. Basis ausbildet, die an mindestens einer Seite mit einem schirmartigen Filterkorb abgeschlossen wird. Diese Ausführungsform führt zu einer besonders guten Filterwirkung.

Mit dem Begriff Filterkorb wird der Teil des Gefäßfilters bezeichnet, der Leiterschleifenwindungen enthält, die dadurch, dass sie an mindestens einer Längsseite des Filters zusammengeführt sind, in der Lage sind, Thromben von geronnenem Blut oder sklerotisches Material im Blutgefäß zurückzuhalten.

In einer weiteren vorteilhaften Ausführungsform, dem sogenannten Diamondfilter, weist der Gefäßfilter mehrere Leiterschleifenwindungen auf, die derart geführt sind, dass der größte Abstand der Schleifenwindungen voneinander längsmittig vorliegt und nach mindestens einer Längsseite hin eine Verjüngung eintritt. In einer besonders vorteilhaften Ausführungsform werden die Leiterschleifenwindungen nach zwei Seiten hin verjüngt, was die Filterwirkung verbessert. Unabhängig von der Anzahl der Filterkörbe liegt der Vorteil dieser Ausführungsform darin, dass ein großer Teil der Leiterschleifenwindungen an der Gefäßwand anliegt, was zu einer guten und dauerhaften und verkippungsfreien Befestigung an der Gefäßwand führt, die zusätzlich durch Verankerung in der Gefäßwand mittels Haken unterstützt werden kann.

Außerdem ermöglicht diese Filterart eine gute Ausleuchtung des Filterinneren im MR-Verfahren.

In einer weiteren vorteilhaften Ausführungsform, die auch als Tulipfilter bezeichnet wird, weist der Gefäßfilter mindestens eine Leiterschleifenwindung auf, die an der einen Seite des Filters in einem Filterkorb zusammenläuft und sich zur anderen Seite des Filters beinartig erstreckt. Besonders bevorzugt ist ein Filter, der mindestens einen Fortsatz ausbildet, der der Verbindung des Filters mit einer Gefäßwand dient. Mit besonderem Vorteil sind die im Fortsatz benachbarten Bereiche der Leiterschleifenwindung in geringem Abstand zueinander geführt. Es ist besonders bevorzugt, dass die im Fortsatz benachbarten Bereiche der Leiterschleifenwindung ohne Zwischenraum miteinander verbunden sind, insbesondere durch Verschweißung, Löten oder Pressen. Sie können aber auch aus einem Stück bestehen, d.h. der Fortsatz ist durch ein einstückiges Teil gebildet, das mit der Leiterschleifenwindung verbunden ist. Dadurch wird die Kontaktfläche des Gefäßfilters mit der Gefäßwand minimiert und erreicht, dass der Filter ohne eine Ruptur der Gefäßwand wieder entfernt werden kann, da die Intima taschenartig um die Fortsätze herum wächst.

Besonders bevorzugt ist es, dass die Fortsätze gegen die Verstrebungen; z. B. des Tulipfilters, verschiebbar angeordnet sind. Die Resonanzfrequenz des Filters hängt auch von seinem Durchmesser ab. Beim Entfalten dieser Ausführungsform des Gefäßfilters verschieben sich die Fortsätze gegen die Verstrebungen in Abhängigkeit vom Gefäßdurchmesser, der die Weite der Entfaltung bestimmt. Durch die Verschiebbarkeit wird die jeweilige Änderung der Resonanzfrequenz mit einer Änderung des Filterdurchmessers kompensiert, so dass die Resonanzfrequenz im Resultat nahezu konstant bleibt. Die Bildgebung im MR bleibt somit unabhängig vom Venendurchmesser optimal. Die Verankerung wird in diesem Fall von der ortsfesten Verstrebung übernommen und ist durch Verankerung mittels Haken möglich.

Besonders bevorzugt ist die Ausbildung eines Doppelfilters. Darunter wird ein Gefäßfilter verstanden, der an den beiden gegenüberliegenden Längsseiten jeweils einen Filterkorb ausbildet. Doppelfilter können beispielsweise in den Ausführungsformen des Umbrella-, Diamond-, und Birdcagefilter realisiert werden. Der Vorteil eines Doppelfilters liegt in einer besseren Ausleuchtung im MR-Bild bei gleichzeitig optimierter Filterwirkung.

Mit Vorteil sind die einzelnen Windungen der Leiterschleife in Längsrichtung des Gefäßfilters angeordnet. Dies führt zu einem mechanisch besonders stabilen Filter und führt außerdem zu einer guten und dauerhaften Verbindung mit der Gefäßwand. Dies ist besonders für einen permanent im Gefäß verbleibenden Gefäßfilter notwendig.

In einer vorteilhaften Ausführungsform weist der erfindungsgemäße Gefäßfilter mindestens eine Verstrebung auf, die mit der Leiterschleife verbunden ist. Diese Verstrebungen verbessern die mechanische Stabilität des Filters und erhöhen die Filterwirkung. Gleichzeitig können sie auch der Verankerung in der Gefäßwand dienen. Die Verstrebungen können an ihren Enden durch Kunststoffverguss oder eine isoliert angebrachte Quetschhülse befestigt sein.

In einer weiteren vorteilhaften Ausführungsform sind die Verstrebungen leitend und leitend mit der Leiterschleife verbunden. Dadurch wird ebenso bei Verwendung des Leiterschleifenmaterials die Herstellung aus nur einem Stück ermöglicht. Somit werden sie Teil der Spule und können die Resonanz verbessern.

In einer besonders bevorzugten Ausführungsform sind die einzelne Verstrebungen verschiebbar mit einzelnen Leiterschleifenwindungen verbunden.

Besonders bevorzugt ist ein Gefäßfilter, der Verstrebungen aus bioresorbierbarem Material enthält.. Nachdem die Verstrebungen von dem biologischen Körper abgebaut worden sind, verfügt der Gefäßfilter über weniger Verbindungen zur Vene oder Arterie als kurz nach der Implantation. Der Gefäßfilter sitzt somit lockerer am Gewebe an. Dies ermöglicht eine leichte Entfernung des Implantates aus dem biologischen Körper.

In einer vorteilhaften Ausführungsform bildet der Gefäßfilter mindestens ein Halbleiterelement, insbesondere eine Diode und/oder ein Transistor und/oder ein integrierter Schaltkreis, aus. Diese Diode ermöglicht die Verstimmung der Resonanzfrequenz, was zu einer besseren Detektierbarkeit in einem MR-System führt. Der Transistor kann ebenfalls den Schwingkreis verstimmen und über eine zusätzliche induktive Schaltspule angesteuert werden.

Mit Vorteil ist die Leiterschleife aus einem Materialstück aus Draht oder elektrische leitendem Kunststoff ausgebildet. Auch eine Ausbildung als Rohr ist möglich. Dies ermöglich eine einfache und kostengünstige Herstellung.

Es ist von Vorteil, die Leiterschleife durch mehrfaches längsweises Schneiden eines Rohres, insbesondere eines Nitinol-Rohres, und anschließendes Aufdehnen herzustellen. Besonders vorteilhaft ist es, die Leiterschleife an den Seiten mäanderförmig zu führen. Durch diese Art der Herstellung wird insbesondere die Isolation der Leiterteile voneinander erleichtert.

Mit Vorteil werden die Enden der Leiterschleifenwindungen durch Verschweißen, Verkleben, Klemmen, Verguss, und/oder Formschluss, insbesondere durch thermisch ausgelöste Schrumpfung eines Zylinders aus Formgedächtnismaterial, zusammengeführt.

In einer weiteren vorteilhaften Ausführungsform enthält der Gefäßfilter mindestens eine Leiterschleifenwindung, die mit mindestens einem Haken versehen ist. Diese Widerhaken ermöglichen eine zusätzliche Befestigung des Filters in der Gefäßwand und verhindern somit eine Filterwanderung.

Mit Vorteil weist der Gefäßfilter mindestens ein Verbindungsmittel, beispielsweise eine Öse, zur Kopplung mit einer Vorrichtung für die Einbringung und/oder Extraktion des Filters auf, beispielsweise einem Katheter. Dies ermöglicht dem Operateur eine leichte Handhabung des Gefäßfilters bei der Implantation und/oder Extraktion.

Weiterhin enthält der Gefäßfilter bevorzugt mindestens ein Mittel zum Bremsen des Filters bei Einbringung in den biologischen Körper. Das Bremsmittel wird bevorzugt durch einen Bereich vergrößerten Durchmessers bzw. durch eine lokale Einengung des Katheter gebildet. Dies ermöglicht dem Operateur eine größere manuelle Kontrolle und somit eine präzisere Implantation in das Blutgefäß.

Besonders bevorzugt ist es, wenn das Verbindungsmittel das Bremsmittel darstellt.

Die Erfindung wird nachfolgend unter Bezugnahme auf die Figuren der Zeichnung anhand mehrerer Ausführungsbeispiele näher erläutert. Es zeigen:
- Fig. 1: eine Darstellung eines Gefäßfilters in der Ausführungsform des Umbrellafilters;
- Fig. 1a: einen Umbrellafilter, der als Doppelfilter ausgebildet ist;
- Fig. 1b: einen Umbrellafilter mit einem Filterkorb;
- Fig. 1c: einen Umbrellafilter mit einem Filterkorb und zwei Dioden;
- Fig. 1d: einen Schaltplan eines Umbrellafilters für das vertikale MRT-Hauptmagnetfeld;
- Fig. 1e: einen Schaltplan eines Umbrellafilters für das vertikale und horizontale MRT-Hauptmagnetfeld;
- Fig. 2: eine Darstellung eines Diamondfilters, der als Doppelfilter ausgebildet ist;
- Fig. 2a: einen Diamondfilter in der Vorderansicht;
- Fig. 2b: einen Diamondfilter in der Seitenansicht;
- Fig. 2c: einen Leiterplan eines Diamondfilters;
- Fig. 2d: einen Querschnitts-Leiterplan eines Diamondfilters;
- Fig. 2e: eine Drahtabwicklung eines Diamondfilters;
- Fig. 2f: Diamondfilter mit einem Verbindungsmittel;
- Fig. 2g: einen Schaltplan eines Diamondfilters für das vertikale und horizontale MRT-Hauptmagnetfeld;
- Fig. 3: die Herstellung eines Gefäßfilters in der Ausführungsform des Diamondfilters;
- Fig. 3a: ein Schnittmuster und einen Leiterbahnwicklungsplan zur Herstellung eines Gefäßfilters aus Nitinol-Rohr;
- Fig. 3b: ein alternatives Schnittmuster und einen Leiterbahnwicklungsplan zur Herstellung eines Gefäßfilters aus Nitinol-Rohr;
- Fig. 3c: ein alternatives Schnittmuster mit zusätzlichen Verstrebungen für die Erhöhung der Filterfunktion des Diamondfilters;
- Fig. 3d: das Schnittmuster der Figur 3c in Seitenansicht im expandierten Zustand;
- Fig. 3e: ein weiteres alternatives Schnittmuster mit zusätzlichen Verstrebungen für die Erhöhung der Filterfunktion des Diamondfilters;
- Fig. 3f: das Schnittmuster der Figur 3e in Seitenansicht um expandierten Zustand;
- Fig. 3g: schematisch ein an einem Schnittmuster angebrachtes Verbindungsmittel;
- Fig. 3h: eine Draufsicht auf ein Nitinol-Rohr mit einem Verbindungsmittel, das gleichzeitig die Funktion des Bremsmittels beim Entlassvorgang aus dem Katheter übernehmen kann;
- Fig. 4: einen Tulipfilter;
- Fig. 4a: einen Tulipfilter in kompakter Form;
- Fig. 4b: einen Tulipfilter mit Fortsätzen, die in Richtung der Filterverjüngung/des Blutstromes zeigen;
- Fig. 4c: einen Tulipfilter mit Verstrebungen, die vom Schwingkreis elektrisch getrennt sind;
- Fig. 4d: einen Schaltplan eines Tulipfilters für das vertikale und horizontale MRT-Hauptmagnetfeld;
- Fig. 5: eine Kopplung zwischen Führungsdraht/Katheter und Gefäßfilter;
- Fig. 5a: eine feste Kopplung zwischen Führungsdraht/Katheter und Gefäßfilter;
- Fig. 5b: eine lösbare Kopplung zwischen Führungsdraht/Katheter und Gefäßfilter;
- Fig. 5c: eine lösbare Kopplung zwischen Führungsdraht/Katheter und Gefäßfilter.

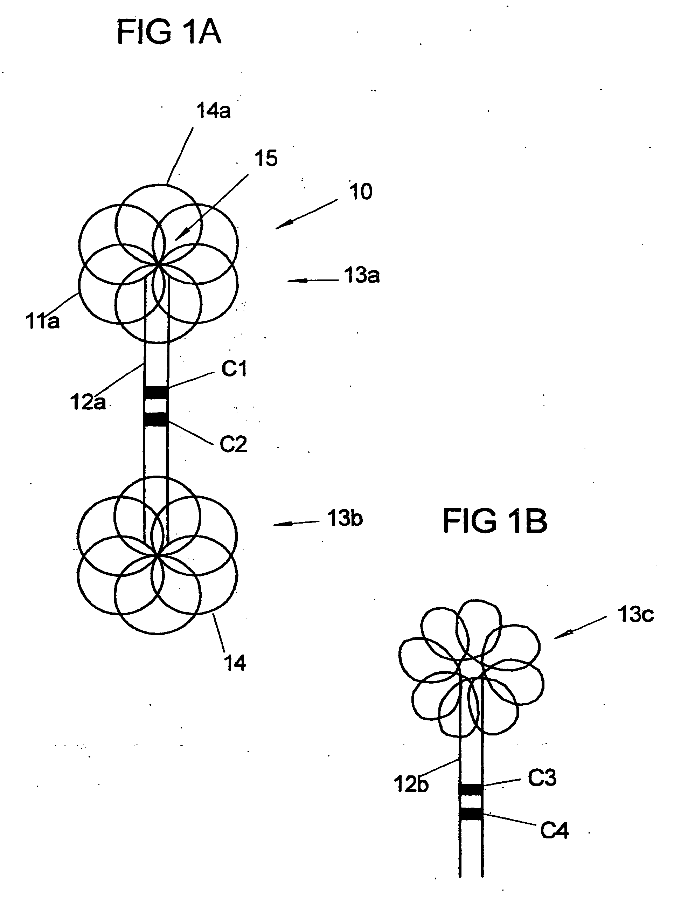

In den Fig. la-le sind Gefäßfilter in der Ausführungsform eines sogenannten Umbrellafilters dargestellt. In Fig. 1a ist der Umbrellafilter 10 als Doppelfilter ausgebildet, wobei eine Leiterschleife 11a eine längliche Basis 12a ausbildet und an beiden Längsseiten der Basis 12a so geführt ist, dass zwei Filterkörbe 13a, 13b ausgebildet sind, die jeweils aus einer Vielzahl, in einem Kreis angeordneter Leiterschleifenwindungen 14, 14a bestehen. In der Mitte der Basis 12a sind zwei Kapazitäten C1, C2 ausgebildet bzw. als gesonderte Teile vorgesehen. Der ganze Gefäßfilter ist, abgesehen von eventuell zusätzlichen Kapazitäten, aus einer Leiterschleife 11a gefertigt.

Im Zentrum 15 der Filterkörbe 13a, 13b sind die einzelnen Leiterschleifenwindungen 14, 14a mittels Isolatoren und Abstandselementen (nicht gesondert dargestellt) voneinander getrennt, um Kurzschlüsse zu vermeiden und eine Resonanzbildung zu ermöglichen.

Andere Geometrien der Filterkörbe sind ebenfalls möglich. So kann die Ausbildung von Kanten die Adaptation an eine Gefäßwand verbessern. Die Filterkörbe 13a, 13b können z. B. als Kreisausschnitte ausgebildet sein. Durch Ausbildung als Dreiecke oder Vielecke kann zudem die Verankerung durch Erhöhung des Druckes auf die Kontaktstellen mit der Gefäßwand verbessern werden.

In Fig. 1b ist ein. Umbrellafilter abgebildet, der nur einen Filterkorb 13c ausbildet. Auch hier sind an der Basis 12b zwei Kapazitäten C3, C4 ausgebildet bzw. als gesonderte Teile vorgesehen.

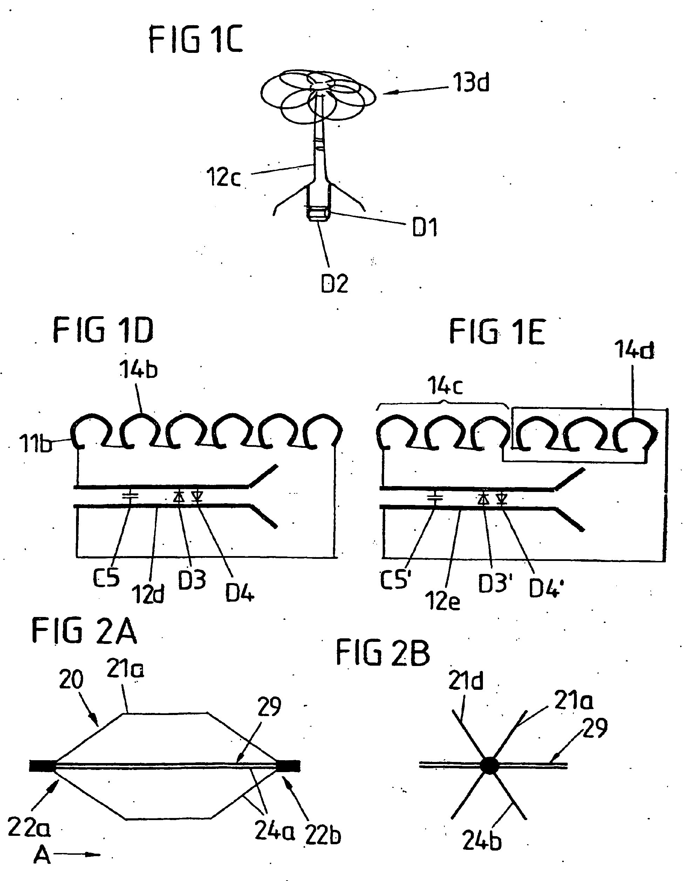

In Fig. 1c sind am Ende der Basis 12c eines. Umbrellafilters an der vom Filterkorb 13d abgewandten Seite zwei Dioden D1, D2 angebracht. Die gegebenenfalls vorhandenen Dioden ermöglichen eine Verstimmung der Resonanzfrequenz, wie in der WO-A1 99/19739 beschrieben.

In Fig. 1d ist ein Schaltplan eines Umbrellafilters gemäß den Figuren 1a-1c gezeigt. Innerhalb der Basis 12d sind sowohl eine Kapazität C5 als auch zwei Dioden (D3, D4) angeordnet. Eine Leiterschleife 11b bildet beispielsweise sechs Leiterschleifenwindungen 14b aus, um den Filterkorb zu bilden. Während in Fig. 1d die Leiterschleife 11b so geführt wird, dass der Filter nur im vertikale MRT-Hauptmagnetfeld aktiv sichtbar ist, so führt die in Fig. 1e gezeigte Verschaltung dazu, dass der Filter sowohl im horizontalen als auch im vertikalen Magnetfeld Resonanz zeigt. Dazu wird nach serieller Verbindung der ersten Leiterschleifenwindungen 14c Kontakt zur letzten Leiterschleifenwindung 14d hergestellt und alle noch nicht verbundenen Leiterschleifenwindungen werden wieder bis zur Mitte seriell miteinander verbunden. Allgemein gesprochen wird für den gleichen Wicklungssinn der Leiterschleifen gesorgt, bezogen auf die verwendete Magnetisierung der Spins. Innerhalb der Basis 12e sind sowohl eine Kapazität C5' als auch zwei Dioden (D3', D4') angeordnet.

So bildet die Leiterschleife eine Induktivität, die mit der Kapazität einen Schwingkreis ausbildet. Der Schwingkreis ist durch das Hochfrequenzfeld eines MR-Bildgebungssystems anregbar, so dass eine verbesserte Darstellung im MR-Bild vorliegt. Die gegebenenfalls vorhandenen Dioden ermöglichen eine Verstimmung der Resonanzfrequenz, wie an sich in der WO-A1 99/19739 beschrieben.

In Fig. 2a ist ein als sogenannter Diamondfilter 20 ausgebildeter Gefäßfilter dargestellt. Der Diamondfilter 20 besteht aus einer Leiterschleife 21a, die mehrere Leiterschleifenwindungen 24a ausbildet. Der größte Abstand der Leiterschleifenwindungen 24a voneinander liegt längsmittig vor, allerdings können einige Leiterschleifenwindungen 24a auch als Doppelwindungen 29 parallel mit geringem Abstand voneinander geführt werden.

Nach beiden Längsseiten hin tritt Verjüngung ein, so dass sich ein Doppelfilter mit zwei seitlichen Filterkörben 22a, 22b ausbildet. Anzumerken ist, dass andere Geometrien der Leiterbahn(en) mit Kanten und Krümmungen möglich sind, um den Kontakt zur Gefäßwand zu verringern und den Druck auf die Gefäßwand durch Verminderung der Anlagefläche zu erhöhen.

In Fig. 2b wird in einer Seitenansicht in Richtung des Pfeils A der Fig. 2a die Leiterschleife 21b, die mehrere Leiterschleifenwindungen 24b ausbildet, dargestellt. Einige Leiterschleifenwindungen 24b werden als Doppelwindungen 29 parallel mit geringem Abstand voneinander geführt. Eine erste Leiterschleife 21a und eine zweite Leiterschleife 21c sind dargestellt. Es sind auch Ausführungsformen möglich, die mehr als eine Leiterschleife enthalten und auch mehr als einen Schwingkreis ausbilden können.

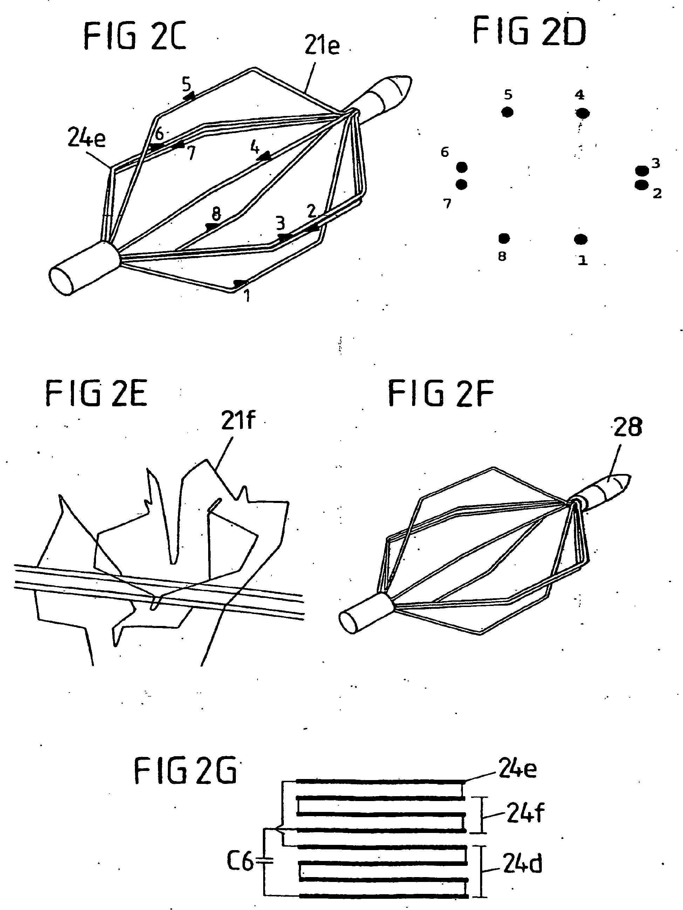

Fig. 2c zeigt die Wicklung der Leiterschleife 21e des Diamondenfilters. Schematisch ist eine Richtung des Wechselstromverlaufs durch Pfeile an den Leiterschleifenwindungen 24c angegeben. In Fig. 2d ist der Diamondfilter der Fig. 2c schematisch in einem Querschnitt entsprechend gezeigt. Der Strom verläuft in den Fig. 2c und 2d angefangen mit 1 hin über 2 zurück, herüber zu 6 hin und über 5 zurück, herüber zu 3 hin und über 4 zurück, herüber zu 8 hin und über 7 zurück.

Der Verlauf kann auch mit weniger Kreuzungspunkten der Leiter mit Vorteil für die Herstellung wie folgt verlaufen: angefangen mit 1 hin über 2 zurück, über 3 hin und über 4 zurück, herüber zu 8 hin und über 7 zurück, über 6 hin und über 5 zurück.

In Fig. 2e ist ein aufgebogener Diamondenfilter abgebildet. Der Filter besteht aus nur einer Leiterschleife 21f, dessen Windungen erkennbar sind.

Fig. 2f zeigt einen Diamondfilter mit einem Verbindungsmittel 28 aus elektrisch leitfähigem Material an einem verjüngten Ende, das ohne Klebeverbindung, z. B. durch Quetschen, Crimpen oder durch thermische Schrumpfung einer Formgedächtnislegierung, befestigt werden kann. Es ist ebenfalls möglich, zwei Verbindungsmittel an den Filter anzubringen. Die Leiterschleifenenden sind mit einem Dielektrikum, insbesondere Epoxydharz, umhüllt, was hier nicht sichtbar ist. Dadurch kommt es zur Ausbildung von Kapazitäten bei der Zusammenführung der Leiterschleifenwindungen.

In Fig. 2g ist ein elektrischer Schaltplan für einen Diamondfilter gezeigt. Es ist eine Kapazität C6 zusammen mit mehreren Leiterschleifenwindungen, in diesem Beispiel acht, zu einem Schwingkreis zusamrnengeschaltet. Nach vier Leiterschleifenwindungen 24d, die seriell geschaltet sind, wird zunächst die Verbindung mit der letzten Leiterschleifenwindung 24e hergestellt. Dann werden seriell bis zum Verzweigungspunkt alle restlichen Leiterschleifenwindungen 24f wieder seriell miteinander verbunden.

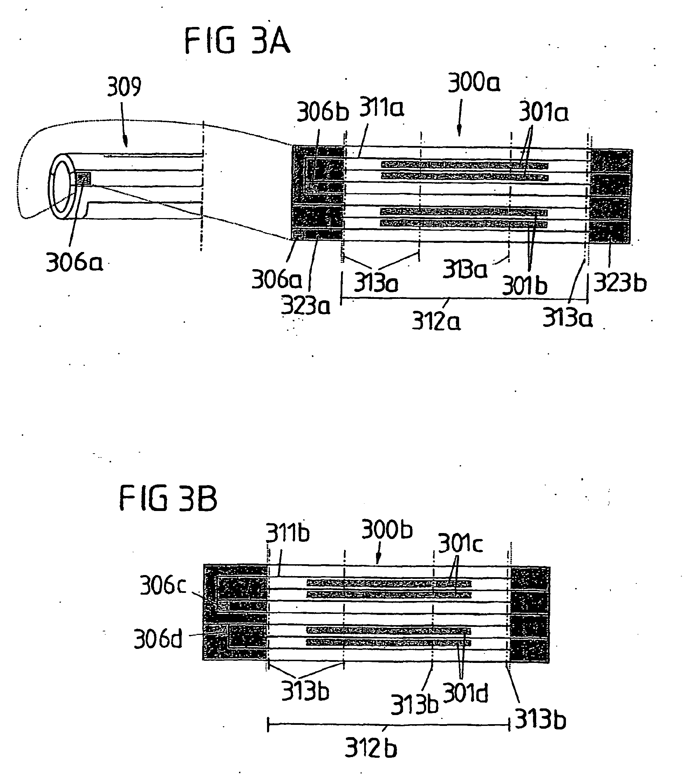

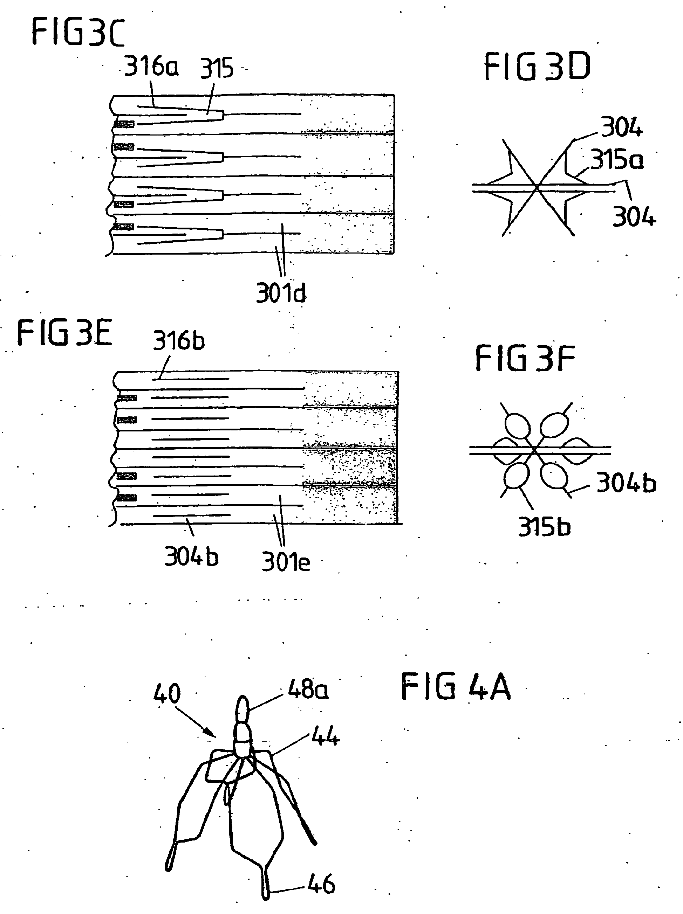

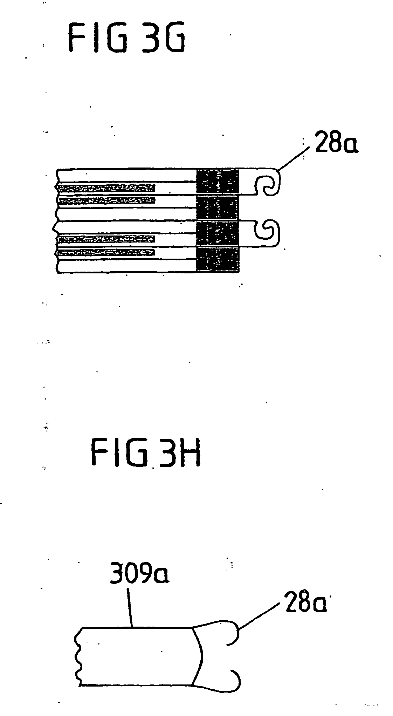

In Fig. 3 wird die Herstellung eines Gefäßfilters in der Ausführungsform des Diamondfilters dargestellt.

In der Fig. 3a ist links ein Nitinol-Rohr 309 mit einem aufgezeichneten Schnittmuster gezeigt. Rechts ist das entsprechende Schnittmuster 300a und der Leiterbahnwicklungsplan zweidimensional wiedergegeben. Das Nitinol-Rohr 309 wird seitlich im Bereich der Zusammenführung bzw. mechanischen Befestigung 323a, b fixiert. Dann wird entlang der Schnittlinien 311a geschnitten und nachfolgend durch Aufdehnen des mittleren Bereichs 312a das geschnittene Nitinol-Rohr 309 aufgedehnt. Das Schnittmuster 300a erlaubt die Fertigung des Gefäßfilters aus nur einem Stück. Das Nitinol-Rohr 309 weist zwei Anschlüsse 306a, 306b für einen Kondensator (nicht gezeigt) auf. Die Biegestellen der Filterstreben 313a sind eingezeichnet. Die paarweise gekennzeichneten Leiterschleifenwindungen 301a sollen nach dem Aufdehnen parallel mit einem Abstand von ca. 1 - 5 mm zueinander beim Aufdehnen verbleiben.

Fig. 3b zeigt je ein alternatives Schnittmuster 300b und 300c bzw. einen Leiterbahnwicklungsplan zur Herstellung eines Gefäßfilters aus Nitinol-Rohr. Wie bereits unter Fig. 3a erläutert, sind die Schnittlinien 311b, der aufzudehnende mittleren Bereich 312b, und die Biegestellen 313b der Leiterschleife gezeigt. Im Gegensatz zu Fig. 3a liegen sich in der Fig. 3b die Anschlüsse 306c und 306d, auf denen ein Kondensator angeschlossen oder ausgebildet werden kann, näher. Statt eines externen Kondensators kann auf den Kondensatoranschlussflächen 306c und 306d auch eine Isolierschicht und darauf eine Leiterschicht aufgetragen werden, die dann elektrischen Kontakt zu einem Ende hat.

In Fig. 3c wird die rechte Hälfte eines Leiterbahnwicklungsplans, ähnlich dem aus Fig. 3b, dargestellt. Ein mögliches Schnittmuster wird gezeigt, welches-die Filterwirkung an mindestens einem der Filterkörbe durch zusätzlich ausgespreizte Filterstreben 315a erhöht. Dabei werden mit einem Laser in jeweils zwei nebeneinander liegende Leiterschleifenwindungen 301d V-förmige Schnitte 316a angebracht, die beim Aufbiegen zu zusätzlichen Verstrebungen 315 von jeweils zwei Leiterschleifenwindungen 301d führen.

Fig. 3d zeigt die expandierte Querschnittsansicht- eines Gefäßfilters. Die Leiterschleifenwindungen 304 werden geteilt und laufen als parallele Leiterschleifenwindungen 315a weiter, ohne sich gegenseitig kurzzuschließen und die Funktion zu gefährden.

Die zusätzlichen Verstrebungen 315a, die jeweils lediglich benachbart der Filterkörbe angeordnet sind, verbessern die Filtereigenschaften hinsichtlich der Filterung des Blutes. Die Verstrebungen können zu einem weiteren gekoppelten oder kombinierten Resonanzschwingkreis gehören.

In Fig. 3e wird, wie schon in Fig. 3c, die rechte Hälfte eines Leiterbahnwicklungsplans ähnlich dem aus Fig. 3b, dargestellt. Ein weiteres mögliches Schnittmuster wird gezeigt, welches die Filterwirkung an mindestens einem der Filterkörbe durch zusätzlich ausgespreizte Filterstreben (315b in Fig. 3f) erhöht. Dabei werden mit einem Laser in jeweils zwei nebeneinander liegende Leiterschleifenwindungen 301e parallel zu den Schnitten, die die Leiterschleifenwindungen erzeugen, zusätzliche Längsschnitte 316b in die einzelnen Windungen eingebracht, jedoch nur in kurze Abschnitte benachbart den Filterkorbenden. Beim Aufbiegen führen diese Schnitte zu zusätzlichen Verstrebungen von jeweils einer Leiterschleifenwindung 304b.

Fig. 3f zeigt die expandierte Längsseitenansicht des Korbes aus Fig. 3e. Die durch die zusätzliche Längsschnitte (316b in Fig. 3e) erzeugten Leiterschleifenwindungen 315b haben nach dem Aufdehnen ovale Form und verbessern die Filtereigenschaften. Sie entstehen durch Teilung der Leiterschleifenwindung 304b und laufen später wieder in selbige zusammen. Runde und eckige Formen sind dabei ebenfalls denkbar. In elektrischer Hinsicht verändern die Aufspreizungen die Homogenität der Felder im Filter, so dass damit die Ausleuchtung im MR-Bild angepasst werden kann.

In Fig. 3g ist ein am Schnittmuster angebrachtes Verbindungsmittel 28a dargestellt. Dieses Verbindungsmittel 28a dient der Kopplung mit einer Vorrichtung für die Einbringung und/oder Extraktion des Filters. Das Verbindungsmittel 28a besteht aus zwei um 180° versetzt angebrachten Haken, die von ein und derselben Seite aus zugänglich sind. Dieses Verbindungsmittel 28a ermöglicht dem Operateur eine leichte Handhabung des Gefäßfilters bei der Implantation und/oder Extraktion. Gleichzeitig hat das Verbindurigsmittel 28a die Funktion eines Bremsmittels. -Durch Bremsen des Filters bei der Einbringung in das Blutgefäß hat der Operateur eine größere manuelle Kontrolle über den Entlassvorgang des Gefäßfilter aus dem Katheter, was letztlich eine präzisere Implantation ermöglicht.

Fig. 3h zeigt in einer Draufsicht ein Nitinol-Rohr 309a entsprechend dem in Fig. 3g dargestellten Schnittmuster. Die beiden gegenüber voneinander angeordneten Teile des Verbindungsmittels 28a sind leicht nach außen gewölbt und dadurch breiter als das Nitinol-Rohr 309a. Beim Herausschieben aus dem Katheter wird ein unkontrolliertes Herausspringen des Gefäßfilter durch die Reibung des Verbindungsmittel an der Katheterinnenwand verhindert.

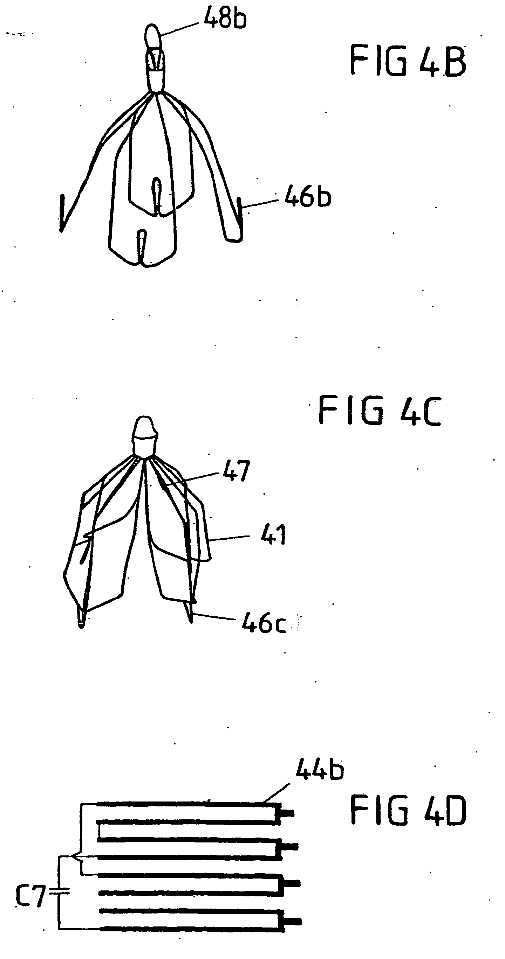

Fig. 4a, 4b und 4c zeigen Ausführungsformen eines sogenannten Tulipfilters 40. In Fig. 4a sind die Leiterschleifenwindungen 44 derart geführt, dass nach mindestens einer Längsseite hin eine Verjüngung eintritt, wobei an der der Verjüngung entgegengesetzten Seite Fortsätze 46 angeordnet sind. Dabei wird es durch z. B. Verschweißen erreicht, dass besagte Fortsätze 46 keine Zwischenräume mehr aufweisen. Ein Verbindungsmittel 48a zum Verbringen in und Entfernen aus dem biologischen Körper ist gezeigt. Wird dieses Verbindungsmittel zuletzt aus einem Katheter entlassen, über den der Venenfilter in den Körper eingebracht wird, hat es gleichzeitig die Funktion eines Bremsmittels, um einen Springeffekt beim Entlassen des Filters zu verhindern. So weist die Öse vor Entfalten des Filters den größten Durchmesser auf.

Die in Fig. 4b gezeigten Fortsätze 46b sind so umgebogen, dass die Enden in Richtung der Filterverjüngung zeigen, also in die Richtung der Blutströmung des' Gefäßes, in dem der Filter angeordnet ist. Nur diese umgebogenen Enden liegen an der Gefäßwand an. Dadurch wird die Kontaktfläche des Gefäßfilters mit der Gefäßwand minimiert und erreicht, dass der Filter ohne eine Ruptur der Gefäßwand wieder entfernt werden kann, da die Intima taschenartig um die Fortsätze herum wächst. Damit wird eine Verankerung in der Gefäßwand und der Intima des Gefäßes erreicht, die der Strömungskraft des Blutes optimal entgegenwirkt. Zur Entfernung des. Gefäßfilters wird der Filter zunächst gegen die Blutstromrichtung bewegt, um die Fortsätze aus den Intimataschen zu entfernen. Nach Zusammenfalten des Filters kann er problemlos wieder aus dem biologischen Körper entfernt werden. Außerdem ist ein Verbindungsmittel 48b zum Verbringen in und Entfernen aus dem biologischen Körper gezeigt, dass wiederum auch als Bremsmittel beim Entlassen dienen kann.

In Fig. 4c sind die Fortsätze 46c verschiebbar zur Leiterschleife 41 angeordnet. Zusätzlich sind Verstrebungen 47 zur Verfestigung des Filterkorbes angeordnet, die vom Schwingkreis elektrisch getrennt sind. Die Fortsätze sind gegen die Verstrebungen 47 des Tulipfilters verschiebbar angeordnet, so dass die Änderung der Resonanzfrequenz durch ein Verschieben bei einer Änderung des Filterdurchmessers kompensiert wird. Die Resonanzfrequenz bleibt dadurch im Resultat im wesentlichen konstant.

Fig. 4d stellt den elektrischen Schaltplan eines Tulipfilters dar. Eine Kapazität C7 ist zusammen mit vier Leiterschleifenwindungen 44b angeordnet, die an einem Ende zusammengeführt werden.

Die Fig. 5a bis 5c zeigen Möglichkeiten der Kopplung zwischen einem Führungsdraht/Katheter einerseits und einem Gefäßfilter andererseits. In Fig. 5a ist eine feste Kopplung zwischen einem Führungsdraht bzw. Katheter 62a und einem Gefäßfilter 63a dargestellt: Dabei wird der Führungsdraht bzw. Katheter 62a aus dem Gefäßfilter 63a herausgeführt.

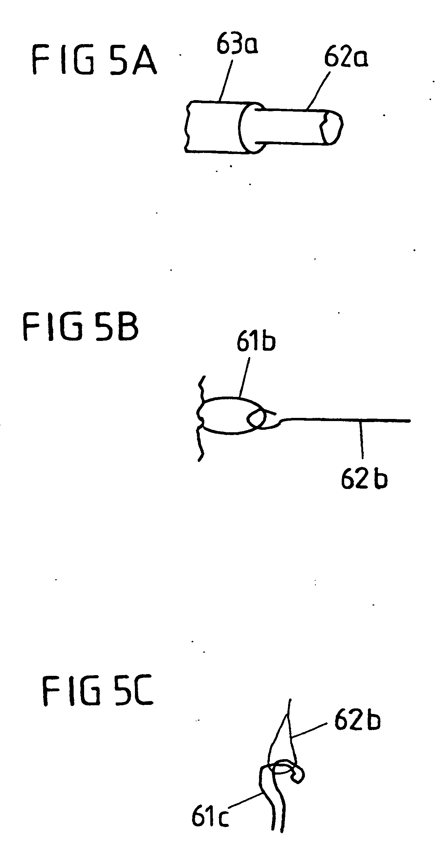

Die Figuren 5b und 5c zeigen demgegenüber Beispiele für lösbare mechanische Kopplungen: Dabei wird die Leiterschleife 61b des Filters in Fig. 5b schlaufenartig aus dem Resonanzschwingkreis herausgeführt. Ein hakenförmiger Führungsdraht bzw. Endbereich eines Katheters 62b kann die Schlaufe erfassen. Wie in Fig. 5c gezeigt, ist es auch möglich, die Schlaufe aus dem Führungsdraht/Katheter 62c und den Haken aus der Leiterschleife 61c auszubilden.

## Patentansprüche

1. Gefäßfilter mit einer Leiterschleife, die die Induktivität eines elektrischen Resonanzschwingkreises ausbildet,
**dadurch gekennzeichnet, dass**
die Leiterschleife (11a, 11b; 21a-f; 41) aus einem Stück besteht und den Gefäßfilter (10; 20; 40) ausbildet.

2. Gefäßfilter nach Anspruch 1, **gekennzeichnet durch** mindestens einen integrierten Schaltkreis, der derart mit dem Resonanzschwingkreis gekoppelt ist, dass dieser durch den integrierten Schaltkreis einstellbar oder verstimmbar ist.

3. Gefäßfilter nach Anspruch 1 oder 2, **dadurch gekennzeichnet, dass** die Leiterschleife (11a, 11b; 21a-f; 41) einzelne Abschnitte (14, 14a-d;.24; 301a-c; 44) und Abstandselemente und/oder Isolatoren aufweist, wobei die Abstandselemente und/oder Isolatoren die einzelne Abschnitte (14, 14a-d; 24; 301a-c; 44) der Leiterschleife in Abstand zueinander halten und/oder gegeneinander isolieren.

4. Gefäßfilter nach mindestens einem der vorhergehenden Ansprüche, **dadurch gekennzeichnet, dass** die Isolatoren in Verbindung mit mindestens einer Leiterschleife (11a, 11b; 21a-f; 41) gleichzeitig eine interne Kapazität ausbilden.

5. Gefäßfilter nach mindestens einem der vorhergehenden Ansprüche, **dadurch gekennzeichnet, dass** die Leiterschleife (11a, 11b; 21a-f; 41) mit einem Nichtleiter, insbesondere Kunststoff und/oder Keramik, ummantelt ist.

6. Gefäßfilter nach Anspruch 5, **dadurch gekennzeichnet, dass** über die Ummantelung eine Kapazität eingestellt ist.

7. Gefäßfilter nach mindestens einem der vorhergehenden Ansprüche, **dadurch gekennzeichnet, dass** der Resonanzschwingkreis eine Resonanzfrequenz, insbesondere im hochfrequenten Bereich besitzt, die der Frequenz eines äußeren Magnetfeldes, insbesondere eines MR-Tomographen, entspricht.

8. Gefäßfilter nach mindestens einem der vorhergehenden Ansprüche, **dadurch gekennzeichnet, dass** die Leiterschleife (11a, 11b; 21a-f; 41) mindestens ein elektrisch nichtleitendes Material aufweist, auf dessen Oberfläche mindestens ein leitendes Material, insbesondere Gold, Platin, Tantal und/oder leitfähige Legierungen, aufgebracht ist.

9. Gefäßfilter nach mindestens einem der vorhergehenden Ansprüche, **dadurch gekennzeichnet, dass** die Leiterschleife (11a, 11b; 21a-f; 41) entfaltbar ist.

10. Gefäßfilter nach Anspruch 9, **dadurch gekennzeichnet, dass** die Leiterschleife (11a, 11b; 21a-f; 41) während und/oder nach der Implantation in einen biologischen Körper entfaltbar ist.

11. Gefäßfilter nach mindestens einem der vorhergehenden Ansprüche, **dadurch gekennzeichnet, dass** die Leiterschleife (11a, 11b; 21a-f; 41) mehrere Leiterschleifenwindungen (14, 14a-d) aüfweist, die derart geführt sind, dass die Leiterschleife (11a, 11b; 21a-f; 41) eine längliche Basis (12a-e) ausbildet, die an mindestens einer Seite mit einem schirmartigen Filterkorb (13a-d) abgeschlossen ist.

12. Gefäßfilter nach mindestens einem der Ansprüche 1 bis 10, **dadurch gekennzeichnet, dass** der Gefäßfilter (20; 40) mehrere Leiterschleifenwindungen (24a-c; 301a-e, 304, 304b; 44) aufweist, die derart geführt sind, dass der größte Abstand der Leiterschleifenwindungen (24a-c; 301a-e, 304, 304b; 44) voneinander in der Mitte des Gefaßfilters (20; 40) vorliegt und an mindestens einer Randseite einen veringeraten Abstand der Leiterschleifenwindungen (24a-c; 301a-e, 304, 304b; 44) voneinander aufweist.

13. Gefäßfilter nach Anspruch 12, **dadurch gekennzeichnet, dass** der Abstand der Leiterschleifenwindungen (24a-c; 301a-e, 304, 304b; 44) voneinander nach zwei Randseiten hin gegenüber der Mitte des Gefäßfilters (20; 40) verringert sind.

14. Gefäßfilter nach mindestens einem -der Ansprüche 1. bis 10, **dadurch gekennzeichnet, dass** der Gefäßfilter (40) mehrere Leiterschleifenwindungen (44) äufweist, die an der einen Seite des Filter in einem Filterkorb (53) zusammenlaufen und sich zur anderen Seite des Filters hin beinartig erstrecken.

15. Gefäßfilter nach mindestens einem der vorhergehenden Ansprüche, **dadurch gekennzeichnet, dass** der Gefäßfilter (40) mindestens eine Leiterschleifenwindung (44) aufweist, die mindestens einen Fortsatz (46) ausbildet, der der Verbindung des Filters mit einer Gefäßwand dient.

16. Gefäßfilter nach Anspruch 15, **dadurch gekennzeichnet, dass** im Fortsatz (46) benachbarte Bereiche der Leiterschleifenwindung (44) in geringem Abstand zueinander geführt sind.

17. Gefäßfilter nach Anspruch 15, **dadurch gekennzeichnet, dass** die im Fortsatz (46) benachbarten Bereiche der Leiterschleifenwindung (44) ohne Zwischenraum miteinander verbunden, insbesondere aus einem Stück hergestellt, verschweißt, verlötet oder verpresst, sind.

18. Gefäßfilter nach mindestens einem der vorhergehenden Ansprüche, **dadurch gekennzeichnet, dass** der Gefäßfilter (-10; 20; 40) einen Doppelfilter ausbildet, wobei Leiterschleifenwindungen (14, 14a) jeweils einen Filterkorb (13a, 13b; 22a, 22b) ausbilden.

19. Gefäßfilter nach mindestens einem der vorhergehenden Ansprüche, **dadurch gekennzeichnet, dass** die Leiterschleife (21a, 21b, 21d-f; 41) einzelne Windungen aufweist, die sich in Längsrichtung des Gefäßfilters (10; 20; 40) erstrecken.

20. Gefäßfilter nach mindestens einem der vorangehenden Ansprüche, **dadurch gekennzeichnet, dass** der Gefäßfilter (10; 20; 40) mindestens eine Verstrebung (47) zur Verfestigung des Gefäßfilters (10; 20; 40) aufweist, die mit der Leiterschleife (11a, 11b; 21a, 21b, 21d-f; 41) verbunden ist.

21. Gefäßfilter nach Anspruch 20, **dadurch gekennzeichnet, dass** die Verstrebungen (47) leitend sind und leitend mit der Leiterschleife (11a, 11b; 21a, 21b, 21d-f; 41) verbunden sind.

22. Gefäßfilter nach Ansprch 20 oder 21, **dadurch gekennzeichnet, dass** einzelne Verstrebungen (47) verschiebbar mit einzelnen Leiterschleifenwindungen (44) verbunden sind.

23. Gefäßfilter nach einem der Ansprüche 15 bis 17 und einem der Ansprüche 20 bis 22; **dadurch gekennzeichnet, dass** der Fortsatz (46) gegen die Verstrebungen (47) verschiebbar angeordnet ist.

24. Gefäßfilter nach einem der Ansprüche 20 bis 23, **dadurch gekennzeichnet, dass** der Gefäßfilter (10; 20; 40) Verstrebungen (47) aus bioresorbierbarem Material enthält.

25. Gefäßfilter nach mindestens einem der vorhergehenden Ansprüche, **dadurch gekennzeichnet, dass** mindestens ein Halbleiterelement, insbesondere eine Diode (D1 bis D4, D3', D4') und/oder ein Transistor und/oder ein integrierter Schaltkreis, am Gefäßfilter (10; 20;. 40) ausgebildet ist.

26. Gefäßfilter nach mindestens einem der vorhergehenden Ansprüche, **dadurch gekennzeichnet, dass** die Leiterschleife (11a, 11b; 21a, 21b, 21d-f; 41) aus einem einzigen Materialstück Draht, oder elektrisch leitfähigem Kunststoff, ausgebildet ist.

27. Gefäßfilter nach mindestens einem der vorhergehenden Ansprüche, **dadurch gekennzeichnet, dass** die eine Leiterschleife (11a, 11b; 21a, 21b, 21d-f; 41) durch mehrfaches längsweises Schneiden eines Röhres (309) und anschließendes Aufdehnen hergestellt ist.

28. Gefäßfilter nach Anspruch 26 oder 27, **dadurch gekennzeichnet, dass** die Leiterschleife (11a, 11b; 21a, 21b, 21d-f; 41) an den Enden des Materialstückes mäanderförmig geführt ist.

29. Gefäßfilter nach mindestens einem der vorhergehenden Ansprüche, **dadurch gekennzeichnet, dass** die Leiterschleifenwindungen (14, 14a-d; 24; 301a-c; 44) an den Enden durch Verschweißen, Verkleben, Klemmen, Verguss, und/oder Formschluss, insbesondere durch thermisch ausgelöste Schrumpfung eines Zylinders aus Formgedächtnismaterial, zusammengeführt sind.

30. Gefäßfilter nach mindestens einem der vorhergehenden Ansprüche, **dadurch gekennzeichnet, dass** mindestens eine Leiterschleifenwindung (14, 14a-d; 24; 301a-c: 44) mit mindestens einem Haken versehen ist ,z. B. einem Verankerungshaken zur Befestigung in einer Gefäßwand.

31. Gefäßfilter nach mindestens einem der vorhergehenden Ansprüche, **dadurch gekennzeichnet, dass** der Gefäßfilter (10; 20; 40) mindestens ein Verbindungsmittel (28, 28a; 48a, 48b) zur Kopplung mit einer Vorrichtung für die Einbringung und/oder Extraktion des Filters aufweist.

32. Gefäßfilter nach mindestens einem der vorhergehenden Ansprüche, **dadurch gekennzeichnet, dass** der Gefäßfilter (10; 20; 40) mindestens ein Mittel (28a) zum Bremsen des Filters bei Einbringung in den biologischen Körper enthält.

33. Gefäßfilter nach den Ansprüchen 31 und 32, **dadurch gekennzeichnet, dass** das Verbindungsmittel (28, 28a; 48a, 48b),so ausgebildet ist, dass es gleichzeitig das Bremsmittel (28a) darstellt.

## Claims

1. Vessel filter comprising a conductor loop that forms the inductance of an electrical resonance circuit,
**characterized in that**
the conductor loop (11a, 11b; 21a-f; 41) is a unitary piece and forms the vessel filter (10; 20; 40).

2. Vessel filter according to claim 1, **characterized by** at least one integrated circuit coupled to the resonance circuit so that it is adjustable or tunable by the integrated circuit.

3. Vessel filter according to claim 1 or 2, **characterized in that** the conductor loop (11a, 11b; 21a-f; 41) comprises individual sections (14, 14a-d; 24; 301a-c; 44) and spacers and/or insulators, in which the spacers and/or insulators keep the individual sections (14, 14a-d; 24; 301a-c; 44) of the conductor loop at a spacing from each other and/or insulate them relative to each other.

4. Vessel filter according to one of the preceding claims, **characterized in that** the insulators simultaneously form an internal capacitance in conjunction with at least one conductor loop (11a, 11b; 21 a-f; 41).

5. Vessel filter according to one of the preceding claims, **characterized in that** the conductor loop (11a, 11b; 21a-f; 41) is coated with a nonconductor, especially plastic and/or ceramic.

6. Vessel filter according to claim 5, **characterized in that** a capacitance is adjusted by the coating.

7. Vessel filter according to one of the preceding claims, **characterized in that** the resonance circuit has a resonance frequency, especially in the high frequency range, that corresponds to the frequency of an external magnetic field, especially of an MR tomograph.

8. Vessel filter according to one of the preceding claims, **characterized in that** the conductor loop (11a, 11b ;21a-f; 41) comprises at least one electrically nonconducting material, on whose surface at least one conductor material, especially gold, platinum, tatalum and/or conducting alloys is applied.

9. Vessel filter according to one of the preceding claims, **characterized in that** the conductor loop (11a, 11b; 21a-f; 41) is deployable.

10. Vessel filter according to claim 9, **characterized in that** the conductor loop (11a, 11b; 21a-f; 41) is deployable during and/or after implantation in a biological body.

11. Vessel filter according to one of the preceding claims, **characterized in that** the conductor loop (11a, 11b; 21a-f; 41) comprises several conductor loop windings (14, 14a-d), guided so that the conductor loop (11a, 11b; 21a-f; 41) forms an elongated base (12a-e), that is sealed on at least one side with a screen-like filter cage (13a-d).

12. Vessel filter according to one of the claims 1 to 10, **characterized in that** the vessel filter (20; 40) comprises a plurality of conductor loop windings (24a-c; 301a-e, 304, 304b; 44) guided so that the greatest spacing of the conductor loop windings (24a-c; 301a-e, 304, 304b; 44) from each other is present in the center of the vessel filter (24; 40) and has a reduced spacing of the conductor loop windings (24a-c; 301a-e, 304b; 44) from each other on at least one edge side.

13. Vessel filter according to claim 12, **characterized in that** the spacing of the conductor loop windings (24a-c; 301a-e, 304b; 44) from each other is reduced toward two edge sides relative to the center of the vessel filter (20; 40).

14. Vessel filter according to one of the claims 1 to 10, **characterized in that** the vessel filter (40) comprises a plurality of conductor loop windings (44) that merge on one side of the filter in a filter cage (53) and extend leg-like to the other side of the filter.

15. Vessel filter according to one of the preceding claims, **characterized in that** the vessel filter (40) comprises at least one conductor loop winding (44) forming at least one extension (46) that serves for connection of the filter to a vessel wall.

16. Vessel filter according to claim 15, **characterized in that** adjacent regions of the conductor loop winding (44) are guided at limited spacing from each other in the extension (46).

17. Vessel filter according to claim 15, **characterized in that** the adjacent regions of the conductor loop winding (44) are connected without intermediate space to each other in the extension (46), especially are produced from one piece, welded, soldered or pressed.

18. Vessel filter according to one of the preceding claims, **characterized in that** the vessel filter (10; 20; 40) forms a double-filter, in which the conductor loop windings (14, 14a) each form a filter cage (13a, 13b; 22a, 22b).

19. Vessel filter according to one of the preceding claims, **characterized in that** the conductor loop (21a, 21b, 21d-f; 41) comprises individual windings that extend in the longitudinal direction of the vessel filter (10; 20; 40).

20. Vessel filter according to one of the preceding claims, **characterized in that** the vessel filter (10; 20; 40) comprises at least one brace (47) for fastening of the vessel filter (10; 20; 40) which is connected to the conductor loop (11a, 11b; 21a, 21b, 21d-f; 41).

21. Vessel filter according to claim 20, **characterized in that** the brace (47) is conducting and connected conducting with the conductor loop (11a, 11b; 21a, 21b, 21d-f; 41).

22. Vessel filter according to claim 20 or 21, **characterized in that** individual braces (47) are movable connected to individual conductor loop windings (44).

23. Vessel filter according to one of the claims 15 to 17 and to one of the claims 20 to 22, **characterized in that** the extension (46) is movably arranged relative to the braces (47).

24. Vessel filter according to one of the claims 20 to 23, **characterized in that** the vessel filter (10; 20; 40) comprises braces (47) made of bioresorbable material.

25. Vessel filter according to one of the preceding claims, **characterized in that** at least one semiconductor element, especially a diode (D1-D4, D3', D4') and/or one transistor and/or one integrated circuit, is formed on the vessel filter (10; 20; 40).

26. Vessel filter according to one of the preceding claims, **characterized in that** the conductor loop (11a, 11b; 21a, 21b, 21d-f; 41) is formed from a single material piece, wire or electrically-conducting plastic.

27. Vessel filter according to one of the preceding claims, **characterized in that** the one conductor loop (11a, 11b; 21a, 21b, 21d-f; 41) is produced by repeated lengthwise cutting of a tube (309) and then expansion.

28. Vessel filter according to claim 26 or 27, **characterized in that** the conductor loop (11a, 11b; 21a, 21b, 21d-f; 41) is guided meander-like on the ends of a single material piece.

29. Vessel filter according to one of the preceding claims, **characterized in that** the conductor loop windings (14, 14a-d; 24; 301a-c; 44) are joined at the ends by welding, gluing, clamping, sealing and/or shape-mating, especially by thermally initiated shrinkage of a cylinder of a shape memory material.

30. Vessel filter according to one of the preceding claims, **characterized in that** at least one conductor loop winding (14, 14a-d; 24; 301a-c; 44) is provided with at least one hook, for example, an anchoring hook, for fastening in a vessel wall.

31. Vessel filter according to one of the preceding claims, **characterized in that** the vessel filter (10; 20; 40) comprises at least one connecting device (28, 28a; 48a, 48b) for coupling to a device for introduction and/or extraction of the filter.

32. Vessel filter according to one of the preceding claims, **characterized in that** the vessel filter (10; 20; 40) comprises at least one means (28a) for braking of the filter during introduction into the biological body.

33. Vessel filter according to claims 31 and 32, **characterized in that** the connection device (28, 28a; 48a, 48b) is constructed and arranged so that it creates simultaneously the braking means (28a).

## Revendications

1. Filtre pour vaisseaux sanguins avec une boucle de conducteurs, développant l'inductance d'un circuit de résonance,
**caractérisé en ce que** la boucle de conducteurs (11a, 11b ; 21 a à f; 41) est en un seul élément et forme le filtre pour vaisseaux sanguins (10 ; 20 ; 40).

2. Filtre pour vaisseaux sanguins selon la revendication 1, **caractérisé par** au moins un circuit intégré qui est couplé de façon telle avec le circuit de résonance, que ce dernier puisse être réglé ou désaccordé par le circuit intégré.

3. Filtre pour vaisseaux sanguins selon la revendication 1 ou 2, **caractérisé en ce que** la boucle de conducteurs (11a, 11b ; 21 a à f ; 41) comporte des sections individuelles (14, 14 a à d ; 24 ; 301 a à c ; 44) et des éléments écarteurs et/ou des isolateurs, les éléments écarteurs et/ou les isolateurs maintenant les sections individuelles (14, 14 a à d ; 24 ; 301 a à c ; 44) de la boucle de conducteurs à distance mutuelle et/ou les isolant mutuellement.

4. Filtre pour vaisseaux sanguins selon au moins l'une des revendications précédentes, **caractérisé en ce que** les isolateurs, en association avec au moins une boucle de conducteurs (11a, 11b ; 21 a à f ; 41) développent simultanément une capacité interne.

5. Filtre pour vaisseaux sanguins selon au moins l'une des revendications précédentes, **caractérisé en ce que** les boucles de conducteurs (11a, 11b ; 21 a à f ; 41) sont enveloppées d'une matière non conductrice, notamment de matière plastique et/ou de céramique.

6. Filtre pour vaisseaux sanguins selon la revendication 5, **caractérisé en ce qu'**une capacité est réglée par l'intermédiaire de l'enveloppe.

7. Filtre pour vaisseaux sanguins selon au moins l'une des revendications précédentes, **caractérisé en ce que** le circuit de résonance a une fréquence de résonance, notamment dans la plage de haute fréquence, qui correspond à la fréquence d'un champ magnétique extérieur, notamment d'un tomographe par RM.

8. Filtre pour vaisseaux sanguins selon au moins l'une des revendications précédentes, **caractérisé en ce que** la boucle de conducteurs (11a, 11b ; 21 a à f ; 41) comporte au moins un matériau non conducteur d'électricité, sur la surface duquel au moins un matériau conducteur, notamment de l'or, du platine, du tantale et/ou des alliages conducteurs est appliqué.

9. Filtre pour vaisseaux sanguins selon au moins l'une des revendications précédentes, **caractérisé en ce que** la boucle de conducteurs (11a, 11b ; 21 a à f ; 41) peut être déployée.

10. Filtre pour vaisseaux sanguins selon la revendication 9, **caractérisé en ce que** la boucle de conducteurs (11a, 11b ; 21 a à f ; 41) peut être déployée pendant et/ou après l'implantation dans un corps biologique.

11. Filtre pour vaisseaux sanguins selon au moins l'une des revendications précédentes, **caractérisé en ce que** la boucle de conducteurs (11a, 11b ; 21 a à f ; 41) comporte plusieurs spires de boucles de conducteurs (14, 14 a à d) qui sont conduites de façon telle, que la boucle de conducteurs (11a, 11b ; 21 a à f ; 41) forme une base oblongue (12 a à e), qui au moins sur un côté se termine par un panier de filtration (13 a à d) du type d'un parapluie.

12. Filtre pour vaisseaux sanguins selon au moins l'une des revendications 1 à 10, **caractérisé en ce que** le filtre pour vaisseaux sanguins (20 ; 40) comporte plusieurs spires de boucle de conducteurs (24 a à c, 301 a à e, 304, 304b ; 44) qui sont conduites de façon telle, que la plus grande distance mutuelle entre les spires de boucle de conducteurs (24 a à c, 301 a à e, 304, 304b ; 44) se trouve au centre du filtre pour vaisseaux sanguins (20 ; 40) et comporte au moins sur un côté marginal une distance réduite entre les spires de boucle de conducteurs (24 a à c, 301 a à e, 304, 304b ; 44).

13. Filtre pour vaisseaux sanguins selon la revendication 12, **caractérisé en ce que** la distance mutuelle entre les spires de boucle de conducteurs (24 a à c, 301 a à e, 304, 304b ; 44) se réduit en direction de deux bords marginaux par rapport au milieu du filtre pour vaisseaux sanguins (20 ; 40).

14. Filtre pour vaisseaux sanguins selon au moins l'une des revendications 1 à 10, **caractérisé en ce que** le filtre pour vaisseaux sanguins (20 ; 40) comporte plusieurs spires de boucle de conducteurs (44), qui sur l'un des côtés du filtre se rencontrent dans un panier de filtration (53) et qui s'étendent comme des branches vers l'autre côté du filtre.

15. Filtre pour vaisseaux sanguins selon au moins l'une des revendications précédentes, **caractérisé en ce que** le filtre pour vaisseaux sanguins (40) comporte au moins une spire de boucle de conducteurs (44) qui forme au moins un prolongement (46) servant à assurer la liaison entre le filtre et une paroi des vaisseau.

16. Filtre pour vaisseaux sanguins selon la revendication 15, **caractérisé en ce que** des zones voisines de la spire de boucle de conducteurs (44) sont conduites à faible distance mutuelle dans le prolongement (46).

17. Filtre pour vaisseaux sanguins selon la revendication 15, **caractérisé en ce que** les zones voisines de la spire de boucle de conducteurs (44) dans le prolongement (44) sont reliées entre elles sans intervalle, qu'elles sont notamment fabriquées, soudées, brasées ou injectées sous pression en monobloc.

18. Filtre pour vaisseaux sanguins selon au moins l'une des revendications précédentes, **caractérisé en ce que** le filtre pour vaisseaux sanguins (10 ; 20 ; 40) forme un double filtre, des spires de boucles de conducteurs (14, 14a) formant respectivement un panier de filtration (13a, 13b ; 22a, 22b).

19. Filtre pour vaisseaux sanguins selon au moins l'une des revendications précédentes, **caractérisé en ce que** la boucle de conducteurs (21a, 21b, 21 d à f ; 41) comporte des spires individuelles, qui s'étendent en direction longitudinale du filtre pour vaisseaux sanguins (10 ; 20 ; 40).

20. Filtre pour vaisseaux sanguins selon au moins l'une des revendications précédentes, **caractérisé en ce que** le filtre pour vaisseaux sanguins (10 ; 20 ; 40) comporte au moins une entretoise (47) pour la consolidation du filtre pour vaisseaux sanguins (10 ; 20 ; 40), qui est reliée avec la boucle de conducteurs (21a, 21b, 21 d à f ; 41).

21. Filtre pour vaisseaux sanguins selon la revendication 20, **caractérisé en ce que** les entretoises (47) sont conductrices et reliées par liaison conductrice avec la boucle de conducteurs (11a, 11 b ; 21a, 21b, 21d à f ; 41).

22. Filtre pour vaisseaux sanguins selon la revendication 20 ou 21, **caractérisé en ce que** des entretoises individuelles (40) sont reliées de façon déplaçable avec des spires individuelles de la boucle de conducteurs (44).

23. Filtre pour vaisseaux sanguins selon l'une quelconque des revendications 15 à 17, et l'une quelconque des revendications 20 à 22, **caractérisé en ce que** le prolongement (46) est disposé de façon déplaçable contre les entretoises (47).

24. Filtre pour vaisseaux sanguins selon l'une quelconque des revendications 20 à 23, **caractérisé en ce que** le filtre pour vaisseaux sanguins (10 ; 20 ; 40) contient des entretoises (47) en matériau biorésorbable.

25. Filtre pour vaisseaux sanguins selon au moins l'une des revendications précédentes, **caractérisé en ce qu'**au moins un élément semi-conducteur, notamment une diode (D1 à D4, D3', D4') et/ou un transistor et/ou un circuit intégré sont conçus sur le filtre pour vaisseaux sanguins (10 ; 20 ; 40).

26. Filtre pour vaisseaux sanguins selon au moins l'une des revendications précédentes, **caractérisé en ce** la boucle de conducteurs (11a, 11b ; 21a, 21b, 21d à f ; 41) est conçu en une pièce de matériau unique, fil métallique ou matière plastique conductrice d'électricité.

27. Filtre pour vaisseaux sanguins selon au moins l'une des revendications précédentes, **caractérisé en ce que** l'une des boucles de conducteurs (11a, 11b ; 21 a, 21b, 21d à f ; 41) est fabriquée par coupes longitudinales multiples d'un tube (309) et par allongement consécutif.

28. Filtre pour vaisseaux sanguins selon la revendication 26 ou 27, **caractérisé en ce que** la boucle de conducteurs (11a, 11b ; 21 a, 21b, 21d à f; 41) est conduite sous forme de méandres sur les extrémités de la pièce de matériau.

29. Filtre pour vaisseaux sanguins selon au moins l'une des revendications précédentes, **caractérisé en ce que** les spires de boucles de conducteurs (14, 14 a à d ; 24 301 a à c ; 44) sont rassemblées sur les extrémités par soudure, collage, serrage; compound et/ou par complémentarité de forme, notamment par rétractation à dédenchement thermique d'un cylindre en matériau à mémoire de forme.

30. Filtre pour vaisseaux sanguins selon au moins l'une des revendications précédentes, **caractérisé en ce qu'**au moins une spire de boucles de conducteurs (14, 14 a à d, 24 ; 301 a à c ; 44) est munie d'au moins un crochet, par exemple d'un crochet d'ancrage pour la fixation dans une paroi de vaisseau sanguin.

31. Filtre pour vaisseaux sanguins selon au moins l'une des revendications précédentes, **caractérisé en ce que** le filtre pour vaisseaux sanguins (10 ; 20; 40) comporte au moins un moyen de liaison (28, 28a ; 48a, 48b) pour le couplage avec un dispositif pour l'introduction et/ou l'extraction du filtre.

32. Filtre pour vaisseaux sanguins selon au moins l'une des revendications précédentes, **caractérisé en ce que** le filtre pour vaisseaux sanguins (10 ; 20; 40) contient au moins un moyen (28a) pour le freinage du filtre lors de l'introduction dans le corps biologique.

33. Filtre pour vaisseaux sanguins selon les revendications 31 et 32, **caractérisé en ce que** le moyen de liaison (28, 28a ; 48a, 48b) est conçu de façon à faire simultanément office de moyen de freinage (28a).
